# EUROPÄISCHE PATENTSCHRIFT

(11) **EP 0 738 385 B1**
(45) Veröffentlichungstag und Bekanntmachung des Hinweises auf die Patenterteilung: **10.07.2002**
(21) Anmeldenummer: 95906915.4
(22) Anmeldetag: 05.01.1995
(51) Int. Cl.: G01N 27/407

(54) **MESSFÜHLER**
MEASURING PROBE
SONDE DE MESURE

(30) Priorität: 05.01.1994 DE 4400220
(43) Veröffentlichungstag der Anmeldung: 23.10.1996
(73) Patentinhaber: EPIQ Sensor-Nite N.V., 3001 Leuven (BE)
(72) Erfinder: HÄFELE, Edelbert, D-76228 Karlsruhe (DE); SEEGER, Walter, D-76571 Gaggenau (DE)
(74) Vertreter: Säger, Manfred, Dipl.-Ing.
(86) Internationale Anmeldenummer: EP9500042
(87) Internationale Veröffentlichungsnummer: WO9518965

(56) Entgegenhaltungen:
- EP-A- 0 015 062
- WO-A-92/08127
- DE-A- 2 702 578
- DE-A- 2 707 062
- DE-A- 4 015 486
- US-A- 4 750 256
- PATENT ABSTRACTS OF JAPAN vol. 12, no. 2 (P-652) 7. Januar 1988 & JP,A,62 163 961 (FUJIKURA LTD) 20. Juli 1987

## Beschreibung

Die Erfindung betrifft einen Meßfühler gemäß dem Oberbegriff des Hauptanspruchs.

Solche Meßfühler sind z.B. als Lambda-Sonden in einer Vielzahl von Ausführungsformen bekannt. Allen Fühlern haftet das Problem an, daß sie einerseits eine Öffnung besitzen müssen, durch die zu messendes Gas eintreten und zu dem eigentlichen Sensor-Chip gelangen kann, andererseits aber gasdicht ausgeführt sein müssen, um zu verhindern, daß dort -wo der Meßfühler, vorzugsweise eingeschraubt ist- das Meßgas austreten kann. Vor allem bereitet aber die mechanische Halterung des Sensor-Chips im heißen sowie pulsierenden Abgas von Verbrennungsmotoren und die thermische Belastbarkeit des elektrischen Anschlusses Probleme.

Der Erfindung liegt deshalb die Aufgabe zugrunde, einen gattungsgemäßen Meßfühler in einfacher Weise mit ausreichender mechanischer sowie thermischer Belastbarkeit auszugestalten.

Diese Aufgabe wird bei einem gattungsgemäßen Meßfühler gemäß dem Oberbegriff des Hauptanspruchs erfindungsgemäß durch dessen kennzeichnende Merkmale gelöst.

Der Meßfühler ist also nach Lehre der Erfindung in einem Gehäuse angeordnet, bei dem der elektrische sowie der mechanische Anschluß des Sensor-Chips, der Keramik, vorzugsweise Al₂O₃ aufweist, durch direkte oder indirekte Laserschweißung mit der bzw. den Kontaktbahnen des Sensor-Chips erfolgt.

Zweckmäßige Ausgestaltung und Weiterbildungen der Erfindung sind in den Unteransprüchen gekennzeichnet.

Die Meßfühler selbst können nach Lehre der Erfindung in einem zylindrischen sowie metallischen Gehäuse angeordnet sein, in das der elektrische Anschluß in Form eines mit einem Metallmantelrohr umschlossenen Innenleiters, nachfolgend kurz Metallmantelleitung genannt, hineingeführt ist, deren Metallmantel mit dem metallischen Gehäuse durch Schweißen oder Löten direkt oder indirekt verbunden ist. Vorzugsweise ist in das rohrförmig ausgebilddete Gehäuse die Metallmantelleitung von einer Seite in das dort offene Rohr hineingeführt. Der elektrische Innenleiter in der Metallmantelleitung kann hierbei in dem Metallmantelrohr in einem elektrischen Isolator, beispielsweise MgO eingebettet sein. Durch das Metallmantelrohr ist eine absolut gasdichte Herausführung der elektrischen Anschlußdrähte aus dem Gehäuse möglich ist. Bei dieser Ausbildung kann der Sensorchip an seinem einen Ende durch den mehr oder weniger starr wirkenden Innenleiter der Metallmantelleitung direkt oder indirekt auch alleine mechanisch gehaltert sein. Durch entsprechende Werkstoffwahl des Innenleiters, z.B. aus einer Kupfer und/oder Aluminium und/oder Eisen und/oder Nickel und/oder Chrom aufweisenden Legierung wird auch eine hohe thermische Belastbarkeit erzielt.

Es ist zwar bekannt, eine Verbindung zwischen einem Anschlußstift und einem Kabel zu dem eigentlichen Sensorelement mittels Laserschweißung durchzuführen (EP-A-0015062). Hierbei wird weder auf einer flachen und dünnen Kontaktbahn lasergeschweißt, wie bei der Erfindung, noch ist diese Kontaktbahn auf einem schlecht wärmeleitenden Keramiksubstrat angeordnet, sondern der Laserstrahl wird auf einen gut wärmeleitenden Stift mit dem eingesetzen Kabel gerichtet, so daß keine Wärmespannungen wie bei der erfindungsgemäßen Lösung zu erwarten sind.

Nach einer besonders bevorzugten Ausführungsform weist der elektrische Anschluß einen Klipp auf, der mechanisch klemmend seitlich sowohl auf die Ober- als auch die Unterseite des Sensor-Chips über dessen Rand an der Längsseite des planar ausgebildeten Sensor-Chips geschoben ist und dort satt und spielfrei auf der Kontaktbahn aufliegt. Der Klipp ist mit der Kontaktbahn des Sensor-Chips laserverschweißt, die vorzugsweise aus einem Edelmetall, z.B. Platin besteht und vermittels eines Haftvermittlers auf dem Substrat des Sensor-Chips aufgebracht ist. Der Haftvermittler weist hierbei ein, vorzugsweise dasselbe Edelmetall wie die Kontaktbahn sowie Glasanteile und/oder Keramik auf. Der Klipp weist ferner eine sich zu der Einsetzöffnung hin erstreckende Anschlußfahne auf, die U-förmig zum Zwecke des thermischen Längenausgleichs abgebogen sowie bandförmig ausgebildet ist. An der Anschlußfahne ist einer der Anschlußdrähte des Innenleiters, vorzugsweise auch durch Laserschweißung angeschlossen. Durch die U-förmige Abbiegung kann im Bereich zwischen den beiden U-Schenkeln ein weiterer Klipp auf eine andere Kontaktbahn des Sensor-Chips aufgesetzt werden, sodaß problemlos vier Klipps eingesetzt werden können.

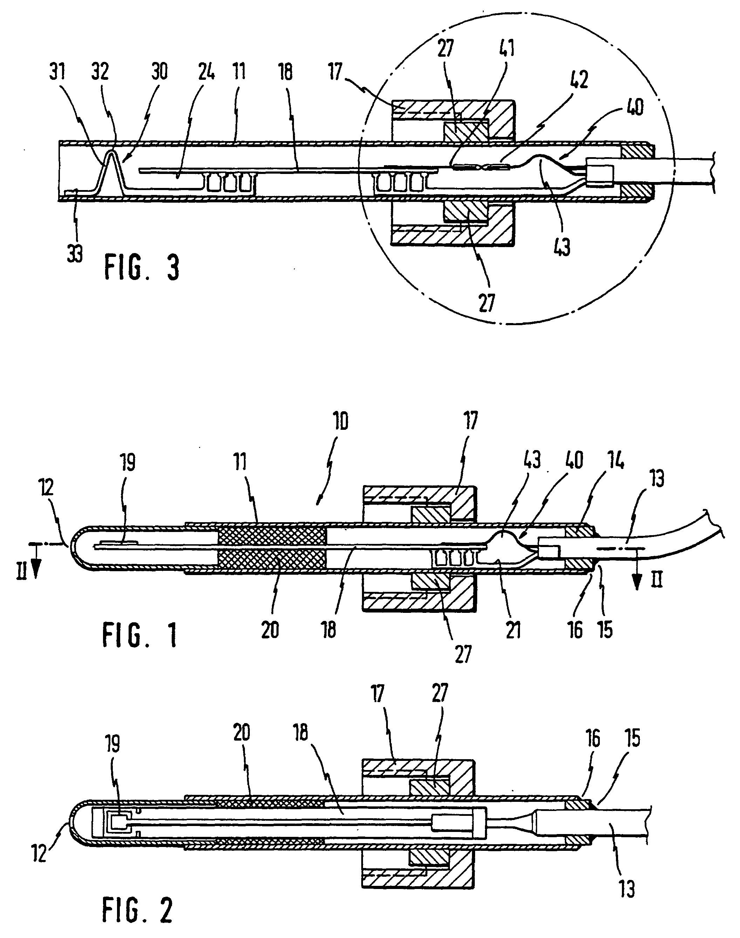

Hierbei können die elektrischen Anschlußdrähte des Innenleiters, die aus der Metallmantelleitung herausgeführt sind, beim offenen System (Fig. 3) dem heißen Abgas, beispielsweise von Verbrennungsmotoren direkt ausgesetzt sein und müssen deshalb entweder aus oberflächen-veredelten Leitungsdrähten oder massiven, oxidationsbeständigen Werkstoffen bestehen. Vorzugsweise wird aber bei der Erfindung ein halboffenes Systemen (Fig. 1 und 2) eingesetzt, bei dem zwischen den elektrischen Anschlußdrähten einerseits und dem eigentlichen, das Sensorelement tragenden Teil des Sensor-Chips ein diesen zusätzlich abstützendes Stützelement vorgesehen ist. Zusätzlich kann das Stützelement, wenn es in bevorzugter Ausgestaltung als vorgeformtes Drahtgestrick ausgebildet ist, auch die in dem Gas vorhandenen Partikel von Schmutz oder dergleichen auffangen, so daß diese nicht in den Bereich der Einsetzöffnung gelangen können.

Bei einer anderen Ausführungsform der Erfindung können die elektrischen Anschlußdrähte im Bogen von der Metallmantelleitung zu dem eigentlichen Sensor-Chip geführt sein und dienen insoweit als Zugkraftausgleich und darüber hinaus zum Ausgleich thermischer Ausdehnungen. Je nach Länge der elektrischen Anschlüsse können diese auch eine hochleitfähige Seele, z.B. aus Kupfer oder Silber aufweisen. Die Verbindung der Anschlußdrähte kann beispielsweise in Form einer Direktkontaktierung dieser Drähte ( gegebenenfalls mit vorheriger Verformung und auch der Anformung des Bogens) direkt mit der Kontaktbahn des Sensor-Chips erfolgen, die aber auch vermittels einer Verbindungshülse angeschlossen sein können. Die direkte Kontaktierung auf dem Substrat erfolgt immer durch eine Laserschweißung. Bei allen andere, z.B. indirekten (auch nicht elektrischen) Kontaktierungen hingegen können auch andere Schweißverfahren angewandt werden. Die Verbindungshülse wird sowohl über die Anschlußfahne als auch über die Enden der elektrischen Anschlußdrähte geführt und kann ebenfalls aus einem hochtemperaturfesten sowie korrosionsfreien Werkstoff bestehen. Die oxidationsanfälligen offenen Enden der beiden Anschlußdrähte werden durch die nachfolgende Schweißung dicht umschlossen. Die Korrosion der offenen Enden der Anschlußleitung kann ebenfalls dadurch unterbunden werden, daß die Schutzhülse der Drahtseele überlappend und formschlüssig bearbeitet wird, so daß sich eine Gasdichtigkeit ergibt. Im Stand der Technik ist die Verbindungsstelle vom eigentlichen Sensor-Chip zum elektrischen Anschluß sowie die Halterung des Sensorelements immer im Bereich eines bereits gasdicht abgetrennten Teils des Gehäuses vorgenommen, wodurch sich für die Abdichtung unter Umständen ein beträchtlicher Aufwand ergibt.

Weiterhin kann der Sensor-Chip auch zum besseren Handling während des Herstellungsprozesses auf seiner einen Seite mit einer Metallisierung versehen, an die ein oder mehrere Füßchen von Beinchen eines Halteteils angeschweißt werden können, die insgesamt über eine Verbindung mit dem in das Innere des Gehäuses des Meßfühlers hereingeführten Metallmantelrohrs verbunden ist, vorzugsweise durch Schweißen. Insoweit ergibt sich bereits im Fertigungsprozeß eine gewisse Stabilität bei den miteinander zu verbindenden Einzelteilen. Außerdem ergibt sich hierbei ein definierter Anker für den eigentlichen Sensor-Chip. Eine Vergrößerung der Länge des Sensor-Chips infolge thermischer Ausdehnung über der kontaktierten Länge läßt sich durch diese Art der rechtwinklig zur Erstreckung des Sensor-Chips verlaufenden Beinchen des Halteteils dadurch ausgleichen, daß sich die Beinchen um einen geringfügigen Winkel verschwenken können. Durch die Beinchen wird die unterschiedliche Ausdehnung über der Länge der Kontaktierung zwischen dem Sensor-Chip und dem Halteteil unter dem Gesichtspunkt von Thermostreß mechanisch entlastet.

Die Beinchen sind hierbei an dem einen Ende des Sensor-Chips angebracht. Dessen anderes Ende kann ebenfalls über eine ein oder mehrere Beinchen aufweisende Verbindung gehaltert sein, indem die am Ende der Beinchen angeordneten Füßchen rechtwinklig von dem eigentlichen Sensor-Chip weggeführt und danach wiederum rechtwinklig in Richtung der Erstreckung des rohrförmigen Gehäuses der Meßfühler weggeführt sind. Über eine insgesamt V-förmige Abbiegung, die an ihrem freien Ende an der Innenseite des Gehäuses festgeschweißt ist, läßt sich einerseits eine feste Halterung des eigentlichen Sensor-Chips erhalten, andererseits ist durch die V-förmige Ausbildung eine gefahrlose thermische Ausdehnung ohne Beschädigung des Sensor-Chips möglich. Beide Halterungen sind hierbei vorzugsweise ebenfalls aus einem hochtemperaturfesten sowie korrosionsbeständigen Werkstoff hergestellt, beispielsweise aus dem gleichen Werkstoff wie das Gehäuse des Sensor-Chips. Beide sich an die Füßchen anschließenden Bereiche der mechanischen Halterung weisen hierbei von der Innenseite des rohrförmigen Metallgehäuses der Meßühler einen Abstand auf. Insgesamt ergibt sich hierbei eine gute mechanische Verbindung zum eigentlichen Sensor-Chip einerseits sowie dem metallischen Gehäuse der Meßfühler andererseits. Werden überdies gleiche Werkstoffe verwendet, ergibt sich mit Vorteil ein gleicher thermischer Ausdehnungskoeffizient. Schließlich ist es möglich, daß die Füßchen nicht nur auf der einen Seite des eigentlichen Sensor-Chips angeordnet sein können, sondern sie können auch dessen Rand zumindest teilweise übergreifen und/oder in Ausnehmungen im Bereich des Randes des Sensor-Chips teilweise eingreifen bzw. übergreifen, wodurch sich durch einen zusätzlichen Form- und/oder Kraftschluß eine insgesamt feste mechanische Halterung ergibt.

Von ganz besonderem Vorteil ist die Tatsache, daß sich praktisch alle Einzelteile, die miteinander zu verbinden sind, durch Laserschweißen verbinden lassen.

Bei den offenen sowie halboffenen Systemen kann die Ansprechzeit des Sensors kann durch die zusätzliche Anbringung von Öffnungen am Umfang des vorderen Bereichs des Gehäuses eingestellt werden. Auch kann der vordere Halter des Sensor-Chips bei der offenen Version gleichzeitig als mechanischer Schutz für das Sensorelement ausgebildet sein. Durch Veränderung der Position des am Gehäuse als Widerlager für eine Überwurfmutter angebrachten Kragens kann die Einbautiefe des Meßfühlers auf einfachste Weise variiert werden.

Zusammenfassend läßt sich also festhalten, daß in bevorzugter Ausführungsform sowohl der elektrische als auch der mechanische Anschluß des Sensor-Chips über die auf dessen Kontaktbahn flächig aufliegenden und in diesem Bereich durch Laserschweißung angeschlossenen Klipps erfolgt, an deren Anschlußfahnen die Anschlußdrähte des Innenleiters der Metallmantelleitung ebenfalls angeschweißt sind, wodurch sich eine besonders einfache und sinnvolle Lösung ergibt.

Ausführungsbeispiele der Erfindung werden nachfolgend unter Bezugnahme auf die Zeichnung näher erläutert. In dieser zeigt:
- Figur 1: einen schematischen Querschnitt durch eine erste Ausführungsform des Meßfühlers;
- Figur 2: einen Schnitt II-II gemäß Fig. 1;
- Figur 3: eine zweite Ausführungsform der Erfindung, und
- Figur 4: eine dritte Ausführungsform der Erfindung, in Draufsicht
- Figur 5: die Ausführungsform gemäß Fig.4, in Seitenansicht
- Figur 6: einen Schnitt VI-VI gemäß Fig.5,
- Figur 7: die Einzelheit VII gemäß Fig. 3 und
- Figur 8: die Einzelheit VIII gemäß Figur 6, schematisch und im im größeren Maßstab.

Die in Fig. 1 insgesamt mit 10 bezeichnete erste Ausführungsform (Fig. 1 und 2) eines Meßfühlers weist ein als zylindrisches Rohr 11 aus einem metallischen Werkstoff gebildetes Gehäuse auf, dessen eine, dem zu messenden Gas zugewandte Seite einen offenen Abschluß mit einer Eintrittsöffnung 12 und an dem anderen Ende einen als Metallmantelleitung ausgebildeten elektrischen Anschluß 13 aufweist, die mit einer aufgesetzten Buchse 14 versehen ist, die einerseits mit mittels einer Laser-Schweißverbindung 15 mit dem äußeren Rohr der Metallmantelleitung und mittels einer Schweißung 16 mit dem offenen Ende des Rohrs 11 verbunden ist.

Im mittleren Bereich ist eine Überwurfmutter 17 vorgesehen, die über ein als Widerlager dienenden Kragen 27 , der ebenfalls durch Schweißen mit dem Rohr 11 verbunden ist, zum Einschrauben des gesamten Meßfühlers in eine Meßöffnung dient.

An dem vorderen Ende des Rohrs 11 ist an seinem der Eintrittsöffnung 12 zugewandten Bereich der das eigentliche Sensorelement 19 des Sensor-Chips 18, der sich in der Rohrlängsrichtung erstreckt, angeordnet. Zwischen dem eigentlichen Sensorelement 19 und dem anschlußseitigen Ende des Sensor-Chips 18 ist ein auf ihn aufgeschobener tragendes Stützteil 20 aus einem entsprechend ge- und/oder vorgeformten Drahtgestrick aufgeschoben. Hierdurch wird der Sensor-Chip in seinem, der Eintrittsöffnung 12 zugewandten Bereich einerseits rechtwinklig zu der Längserstreckung des Rohrs mechanisch gehaltert. Andererseits können in dem Abgas enthaltenen Partikelchen etc. nicht bis in den Bereich des elektrischen Anschlusses der Metallmantelleitung 13 vordringen.

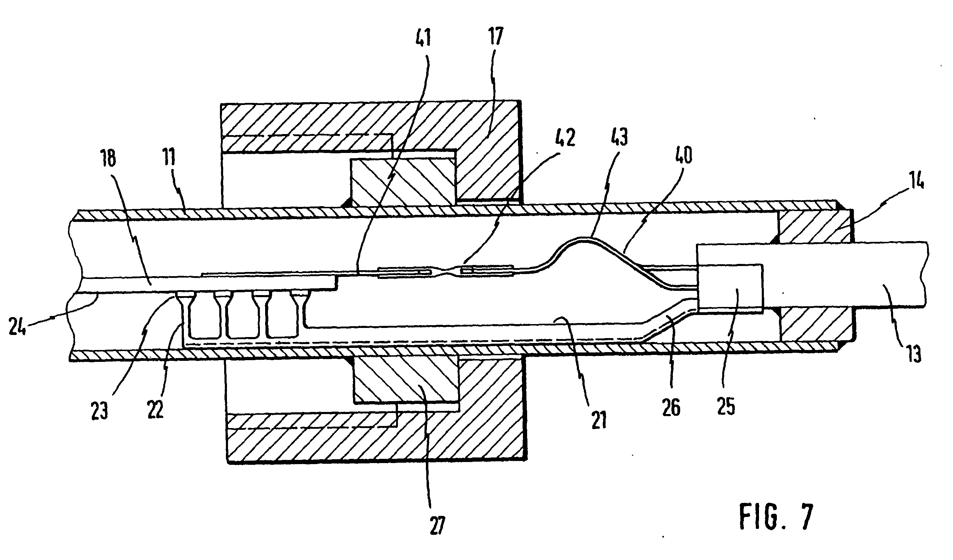

Auf dem dem eigentlichen Sensorelement 19 abgewandten Bereich des Sensor-Chips 18 ist bei der ersten sowie zweiten (Fig. 3 und 7) Ausführungsform ein insgesamt mit 21 bezeichnetes Halteteil vorgesehen. Dieses Halteteil hat eine im wesentlichen parallel zur Längserstreckung des Rohrs 11 verlaufende Erstreckung. Rechtwinklig hiervon stehen Beinchen 22 ab, die an ihren Enden mit Füßchen 23 (Fig. 7) versehen sind. Die - in Fig. 7 untere - Seite des Sensor-Chips 18 weist eine insgesamt mit 24 bezeichnete Metallisierung auf, an die die Füßchen 23 des insgesamt mit 21 bezeichneten Halters angeschweißt sind. Außerdem kann das Halteteil 21 mit einem über eine Abbiegung 26 abgebogenen, teilzylindermantelförmigen Umfassungsstück 25, ebenfalls durch Schweißen mit dem äußeren Rohr der Metallmantelleitung 13, ebenfalls durch Schweißen oder Löten verbunden sein.

Anstelle des Drahtgestricks 20 kann bei der zweiten Ausführungsform gemäß Figur 3 im vorderen Teil ein in etwa dem Halteteil 21 entsprechendes Halteteil 30 vorgesehen sein, das ebenfalls quer zu seiner Längserstreckung quer, vorzugsweise rechtwinklig verlaufende Beinchen mit an dessen Ende vorgesehenen Füßchen versehen ist, die an der Metallisierung 24 mit der Unterseite des Sensor-Chips 18 durch Laserschweißen verbunden sind. Dieses Halteteil 30 ist mit einer V-förmigen Abbiegung 31 versehen, deren Spitze 32 von der gegenüberliegenden Innenseite des Rohrs 11 einen Abstand aufweist. Das äußerste Ende 33 dieses Halteteils 30 ist an der Innenseite des Rohrs 11, beispielsweise durch Schweißen festgelegt. Die unterschiedliche thermische Längenausdehnung zwischen dem Rohr 11 und der gesamten Länge des Sensor-Chips 18 wird durch die V-förmige Abbiegung 31 ausgeglichen.

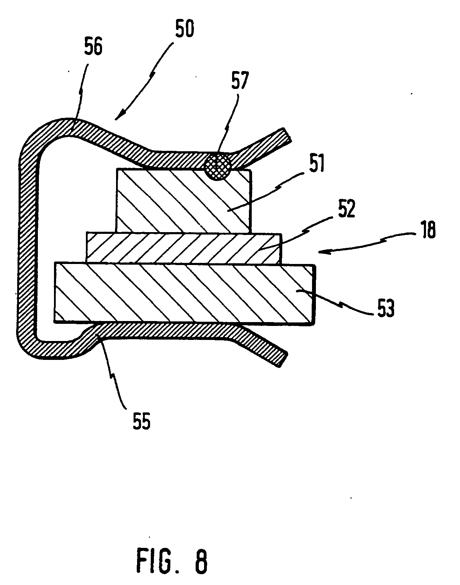

Der elektrische Anschluß des das eigentliche Sensorelement 19 tragenden Sensor-Chips 18 kann entweder dadurch erfolgen, daß die Anschlußdrähte 40 der Metallmantelleitung 13 direkt auf den Sensor-Chip aufgeschweißt (Fig. 1), oder aber vermittels an dem Sensor-Chip 18 angebrachter Anschlußfahnen 41 (Fig. 3,4 bis 6 sowie 8) und gegebenenfalls einer Verbindungshülse 42 elektrisch miteinander verbunden sind. In beiden Fällen sind die Anschlußdrähte so vorgeformt (z.B. in Form eines als Zugentlastung dienenden Bogens 43 geführt sein) und im Anschlußbereich zum Substrat quer zur Längserstreckung so verformt sein, daß sich ein zumindest bereichsweise flächiges Auflager ergibt.

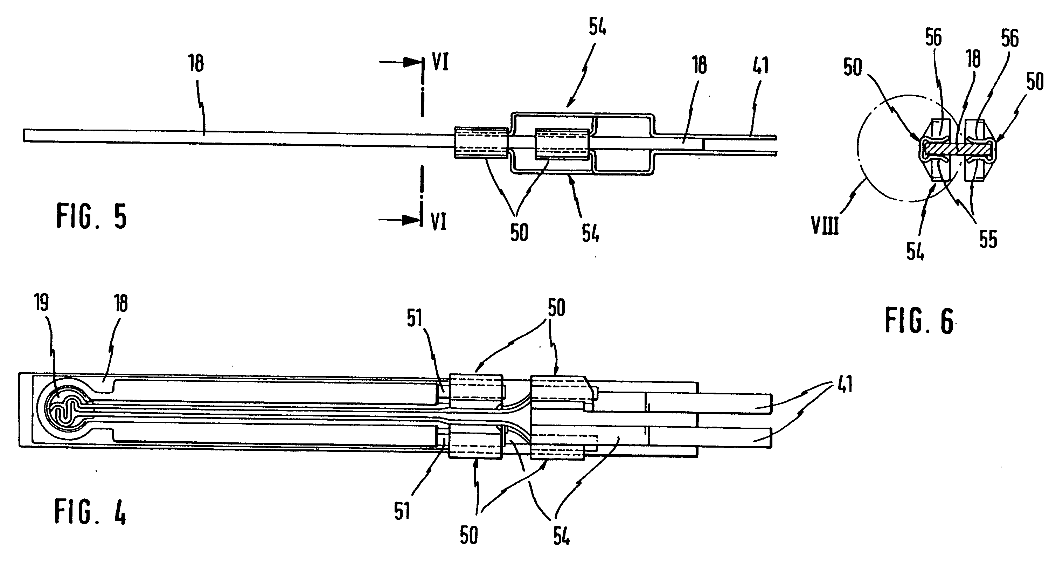

Eine dritte, ganz bevorzugte Ausführungsform mit einem elektrisch leitenden Klipp 50 ist in den Figuren 4 bis 6 (ohne das Gehäuse) sowie 8 als halboffenes System mit dem tragenden Stützteil 20 dargestellt und weist den Vorteil auf, daß der Klipp eine Doppelfunktion, nämlich sowohl als elektrischer Anschluß als auch der mechanischen Halterung aufweist. Der Klipp weist eine eine U-förmige Abbiegung 54 aufweisende Anschlußfahne 41 auf, die überdies bandförmig ausgebildet ist, und ist über eine Längskante des planaren Sensor-Chips 18 so aufgesetzt, daß die beiden federnden Arme 55, 56 der Klipps 50 satt und flächig sowohl auf der Ober- als auch der Unterseite des Sensor-Chips aufliegen.

Hinter der U-förmigen Abbiegung 54 beginnt die eigentliche Anschlußfahne 41, welche beim wiedergegebenen Ausführungsbeispiel wieder auf der Oberfläche des Sensor-Chips 18 aufliegt, wodurch die Steifigkeit dieser Anordnung vergrößert wird. An dieser Anschlußfahne 41 wird ein Anschlußdraht 40 des Innenleiters der Metallmantelleitung 13 angeschlossen, vorzugsweise durch Laserschweißung. Im Bereich der U-förmigen Abbiegung 54 des einen Klipps können die beiden Arme eines anderen Klipps seitlich aufgesteckt sein, dessen U-förmige Abbiegung auf der anderen Seite des Sensor-Chips angeordnet ist.

Auf dem Substrat 53 , z.B. Al₂O₃ des Sensor-Chips 18 ist ferner eine Haftvermittlungsschicht 52 durch Siebdruck und auf dieser, ebenfalls siebgedruckt eine Platin aufweisende Schicht als Kontaktbahn 51 aufgebracht. Auf dieser liegt der eine Arm 56 des Klipp 50 auf, der mittels Laserschweißung mit der Kontaktbahn 51 verschweißt ist, wie schematisch mit 57 bezeichnet.

## Patentansprüche

1. Meßfühler (10) für ein Meßgas mit einem metallischen Gehäuse (11), mit einem darin angeordneten, an seinem einen, einer Eintrittsöffnung (12) des Gehäuses (11) für das Meßgas zugewandten Endbereich das Sensorelement (19) aufweisenden Sensor-Chip (18) und mit einem an dessen anderem Ende vorgesehenen, mit dem Sensorelement (19) elektrisch verbundenen, elektrischen Anschluß, **dadurch gekennzeichnet, daß** der Sensor-Chip (18) Keramik als Substrat (53) aufweist, daß darauf zumindest eine zu dem Sensor-Element (19) führende sowie elektrisch leitende Kontaktbahn (51) als Schicht vorgesehen ist und daß der elektrische und mechanische Anschluß mit der Kontaktbahn (51) durch direkte kraftschlüssige Laserschweißung zumindest eines Anschlußdrahtes (40) auf die Kontaktbahn (51) erfolgt.

2. Meßfühler (10) für ein Meßgas mit einem metallischen Gehäuse (11), mit einem darin angeordneten, an seinem einen, einer Eintrittsöffnung (12) des Gehäuses (11) für das Meßgas zugewandten Endbereich das Sensorelement (19) aufweisenden Sensor-Chip (18) und mit einem an dessen anderem Ende vorgesehenen, mit dem Sensorelement (19) elektrisch verbundenen, elektrischen Anschluß, **dadurch gekennzeichnet, daß** der Sensor-Chip (18) Keramik als Substrat (53) aufweist, daß darauf zumindest eine zu dem Sensor-Element (19) führende sowie elektrisch leitende Kontaktbahn (51) als Schicht vorgesehen ist und daß der elektrische und mechanische Anschluß mit der Kontaktbahn (51) indirekt kraftschlüssig durch Laserschweißung eines Klipps (50) erfolgt, wobei ein Anschlußdraht (40) mit dem Klipp (50) verschweißt oder verlötet und dieser mit der Kontaktbahn (51) des Sensor-Chips (18) laserverschweißt ist.

3. Fühler nach einem der Ansprüche 1 oder 2, **dadurch gekennzeichnet, daß** der Fühler einen Klipp (50) aufweist und daß der Innenleiter (40) mit dem Klipp (50) verschweißt oder verlötet und dieser mit der Kontaktbahn (51) des Sensorchips (18) laserverschweißt ist.

4. Fühler nach einem der Ansprüche 1 bis 3, **dadurch gekennzeichnet, daß** der beide Seiten des Sensor-Chips (18) seitlich umgreifende, elektrisch leitende Klipp (50) auf eine der Kontaktbahnen (51) des Sensor-Chips (18) aufgesetzt und in dem Bereich dieser Kontaktbahn lasergeschweißt ist.

5. Fühler nach einem der Ansprüche 1 bis 4, **dadurch gekennzeichnet, daß** der Klipp (50) zwei federnde Arme (55, 56) aufweist, die zumindest in einem Teilbereich der Kontaktbahn (51) flächig aufliegen und mit dieser in diesem flächigen Auflagebereich durch Laserschweißung verbunden sind.

6. Fühler nach einem der Ansprüche 1 bis 5, **dadurch gekennzeichnet, daß** jeder Klipp (50) einer sich zu der Einsetzöffnung hin erstreckende , vorzugsweise eine U-förmige Abbiegung (54) aufweisende Anschlußfahne (41) aufweist, an der einer der Anschlußdrähte des Innenleiters (40) der Metallmantelleitung (13) angeschweißt ist.

7. Fühler nach einem der Ansprüche 1 bis 6, **dadurch gekennzeichnet, daß** der vorzugsweise länglich bzw. planar ausgebildete Sensor-Chip (18) zwischen dem Sensorelement (19) und der Einsetzöffnung mindestens ein ihn nur rechtwinklig zu der Längserstreckung des Sensor-Chips (18) stützendes Stützelement (20, 30) aufweist.

8. Fühler nach Anspruch 7, **dadurch gekennzeichnet, daß** das Stützelement als auf den Sensor-Chip (18) aufgesetztes, vorzugsweise vorgeformtes Drahtgestrick (20) ausgebildet ist.

9. Fühler nach einem der Ansprüche 1 bis 8, **dadurch gekennzeichnet, daß** das metallische Gehäuse als zylindrisches Rohr (11) ausgebildet ist, dessen eines Ende die Eintrittsöffnung (12) für das mittels des Sensorelements (19) zu messende Abgas aufweist und dessen anderes offenes Ende als Einsetzöffnung für die eingesetzte und dort geschweißte oder gelötete Metallmantelleitung (13) ausgebildet ist.

10. Fühler nach Anspruch 9, **dadurch gekennzeichnet, daß** auf die Metallmantelleitung (13) eine metallische Buchse (14) aufgesetzt sowie in diesem Zustand angelötet oder -geschweißt ist, und daß diese ihrerseits an dem Rohr (11) angelötet oder angeschweißt ist.

11. Fühler nach einem der Ansprüche 1 bis 10, **dadurch gekennzeichnet, daß** der Sensor-Chip (18) auf seiner einen Seite eine zum mechanischen Anschluß als Metallisierung (24) ausgebildete Kontaktbahn aufweist, an die zumindest ein im wesentlichen senkrecht darauf stehendes Beinchen (22) eines Halteteils (21) mit seinen am Ende der Beinchen angeordneten Füßchen (23) mittels Laserschweißung angeschweißt ist.

12. Fühler nach einem der Ansprüche 1 bis 11, **dadurch gekennzeichnet, daß** das Halteteil sich in Richtung des Rohrs (11) erstreckt und daß sich die Beinchen (22) quer, vorzugsweise rechtwinklig von dem Halteteil (21) erstrecken.

13. Fühler nach Anspruch 9, 10, 11 oder 12 , **dadurch gekennzeichnet, daß** das Halteteil (30) sich in Richtung des Rohrs (11) erstreckt und über eine V-förmige Abbiegung (31) mit seinem äußersten Ende (33) an der Innenseite des Rohrs (11) festgelegt, vorzugsweise angeschweißt ist (Fig.3).

14. Fühler nach Anspruch 13, **dadurch gekennzeichnet, daß** die V-förmige Abbiegung (31) zwischen dem eigentlichen Sensorelement (19) und der Eintrittsöffnung (12) angeordnet ist.

15. Fühler nach einem der Ansprüche 1 bis 14, **dadurch gekennzeichnet, daß** zumindest eines der Beinchen (22) über eine Verbindung (26) mit dem Metallmantel der Metallmantelleitung (13) oder der Buchse (14) mit einem Umfassungsteil (25) verbunden, vorzugsweise angeschweißt ist.

16. Fühler nach einem der Ansprüche 1 bis 15, **dadurch gekennzeichnet, daß** die Beinchen (22) den Rand des Sensor-Chips (18) zumindest teilweise übergreifen und/oder in Ausnehmungen im Bereich des Randes desselben (18) eingreifen.

17. Fühler nach einem der Ansprüche 1 bis 16, **dadurch gekennzeichnet, daß** zumindest eines der Beinchen (22) zweimal rechtwinklig in Richtung auf die andere, die Metallisierung (24) nicht aufweisende Seite des Sensor-Chips (18) abgebogen ist.

18. Fühler nach einem der Ansprüche 1 bis 17, **dadurch gekennzeichnet, daß** die einzelnen elektrischen Anschlußdrähte des Innenleiters (40) der Metallmantelleitung (13) des elektrischen Anschlusses in dem, dem zu messende Abgas ausgesetzten Innenraum des Gehäuses miteinander verbunden sind.

19. Fühler nach Anspruch 18, **dadurch gekennzeichnet, daß** die elektrischen Anschlußdrähte des Innenleiters (40) direkt auf den Sensor-Chip, gegebenenfalls mittels eines vorgeformten Bogens (43) als Längenausgleich und/oder Zugentlastung direkt auf den Sensor-Chip (18) durch Laserschweißung angeschweißt sind.

20. Fühler nach einem der Ansprüche 1 bis 19, **dadurch gekennzeichnet, daß** die Anschlußdrähte (40) über eine Verbindungshülse (42) mit den Anschlußfahnen (41) verbunden sind.

21. Fühler nach einem der Ansprüche 1 bis 20, **dadurch gekennzeichnet, daß** die Anschlußdrähte (40) für ihren Anschluß vorgeformt, beispielsweise in Form eines Bogens (43) vorgeformt sind.

22. Fühler nach einem der Ansprüche 1 bis 21, **dadurch gekennzeichnet, daß** die Kontaktbahn (51) über einen Haftvermittler (52) mit dem Substrat (53) des Sensor-Chips (18) verbunden ist.

23. Fühler nach Anspruch 22, **dadurch gekennzeichnet, daß** die Kontaktbahn (51) mittels Siebduck aufgebracht ist.

24. Fühler nach Anspruch 22, **dadurch gekennzeichnet, daß** der Haftvermittler (52) mittels Siebduck aufgebracht ist.

25. Fühler nach einem der Ansprüche 22 bis 24, **dadurch gekennzeichnet, daß** die Kontaktbahn (51) Platin aufweist.

26. Fühler nach einem der Ansprüche 22 bis 24, **dadurch gekennzeichnet, daß** der Haftvermittler (52) neben Platin auch Glas und/oder Keramik aufweist.

## Claims

1. A measuring probe (10) for a gas to be measured comprising a metal casing (11), a sensor chip (18) which is arranged therein and which has the sensor element (19) at its one end region which is towards an entry opening (12) of the casing (11) for the gas to be measured, and an electrical connection which is provided at the other end thereof and which is electrically connected to the sensor element (19), **characterised in that** the sensor chip (18) has ceramic as the substrate (53), that provided thereon is at least one contact track (51) in the form of a layer, which is electrically conductive and leads to the sensor element (19), and that electrical and mechanical connection to the contact track (51) is effected by direct force-locking laser welding of at least one connecting wire (40) on to the contact track (51).

2. A measuring probe (10) for a gas to be measured comprising a metal casing (11), a sensor chip (18) which is arranged therein and which has the sensor element (19) at its one end region which is towards an entry opening (12) of the casing (11) for the gas to be measured, and an electrical connection which is provided at the other end thereof and which is electrically connected to the sensor element (19), **characterised in that** the sensor chip (18) has ceramic as the substrate (53), that provided thereon is at least one contact track (51) in the form of a layer, which is electrically conductive and leads to the sensor element (19), and that electrical and mechanical connection to the contact track (51) is effected indirectly in force-locking relationship by laser welding of a clip (50), wherein a connecting wire (40) is welded or soldered to the clip (50) and same is laser-welded to the contact track (51) of the sensor chip (18).

3. A probe according to one of claims 1 and 2 **characterised in that** the probe has a clip (50) and that the internal conductor (40) is welded or soldered to the clip (50) and same is laser-welded to the contact track (51) of the sensor chip (18).

4. A probe according to one of claims 1 to 3 **characterised in that** the electrically conducting clip (50) which laterally embraces both sides of the sensor chip (18) is fitted on to one of the contact tracks (51) of the sensor chip (18) and is laser-welded in the region of said contact track.

5. A probe according to one of claims 1 to 4 **characterised in that** the clip (50) has two resilient arms (55, 56) which at least in a portion of the contact track (51) bear flat thereagainst and are connected thereto in said flat contact region by laser welding.

6. A probe according to one of claims 1 to 5 **characterised in that** each clip (50) has a connecting lug (41) which extends towards the insertion opening and which preferably has a U-shaped bend (54) and to which one of the connecting wires of the internal conductor (40) of the metal sheath line (13) is welded.

7. A probe according to one of claims 1 to 6 **characterised in that** the sensor chip (18) which is preferably of an elongate or planar configuration, between the sensor element (19) and the insertion opening, has at least one support element (20, 30) which supports it only at a right angle relative to the longitudinal extent of the sensor chip (18).

8. A probe according to claim 7 **characterised in that** the support element is in the form of a preferably pre-shaped wire mesh (20) which is fitted on to the sensor chip (18).

9. A probe according to one of claims 1 to 8 **characterised in that** the metal casing is in the form of a cylindrical tube (11) of which one end has the entry opening (12) for the exhaust gas which is to be measured by means of the sensor element (19) and whose other open end is in the form of an insertion opening for the inserted metal sheath line (13) which is welded or soldered there.

10. A probe according to claim 9 **characterised in that** a metal sleeve (14) is fitted on to the metal sheath line (13) and soldered or welded thereto **in that** condition and that it is in turn soldered or welded to the tube (11).

11. A probe according to one of claims 1 to 10 **characterised in that** on its one side the sensor chip (18) has a contact track which for mechanical connection purposes is in the form of a metallisation (24) and to which at least one leg portion (22), which is disposed substantially perpendicularly thereto, of a holding member (21) is welded with its foot portion (23) arranged at the end of the leg portion, by means of laser welding.

12. A probe according to one of claims 1 to 11 **characterised in that** the holding member extends in the direction of the tube (11) and that the leg portions (22) extend transversely and preferably at a right angle from the holding member (21).

13. A probe according to claim 9, claim 10, claim 11 or claim 12 **characterised in that** the holding portion (30) extends in the direction of the tube (11) and is fixed and preferably welded by way of a V-shaped bend (31) with its outermost end (33) to the inside of the tube (11) (Figure 3).

14. A probe according to claim 13 **characterised in that** the V-shaped bend (31) is arranged between the actual sensor element (19) and the entry opening (12).

15. A probe according to one of claims 1 to 14 **characterised in that** at least one of the leg portions (22) is connected and preferably welded by way of a connection (26) to the metal sheathing of the metal sheath line (13) or the sleeve (14) with an embracing portion (25).

16. A probe according to one of claims 1 to 15 **characterised in that** the leg portions (22) at least partially engage over the edge of the sensor chip (18) and/or engage into openings in the region of the edge thereof (18).

17. A probe according to one of claims 1 to 16 **characterised in that** at least one of the leg portions (22) is bent over twice at a right angle towards the other side of the sensor chip (18), which side does not have the metallisation (24).

18. A probe according to one of claims 1 to 17 **characterised in that** the individual electrical connecting wires of the internal conductor (40) of the metal sheath line (13) of the electrical connection are connected together in the interior of the casing, which is exposed to the exhaust gas to be measured.

19. A probe according to claim 18 **characterised in that** the electrical connecting wires of the internal conductor (40) are welded directly on to the sensor chip, possibly by means of a pre-shaped curve (43) as length compensating means and/or tensile load relief means directly on to the sensor chip (18) by laser welding.

20. A probe according to one of claims 1 to 19 **characterised in that** the connecting wires (40) are connected to the connecting lugs (41) by way of a connecting sleeve (42).

21. A probe according to one of claims 1 to 20 **characterised in that** the connecting wires (40) are pre-shaped for the connection thereof, preferably being pre-shaped in the form of a curve (43).

22. A probe according to one of claims 1 to 21 **characterised in that** the contact track (51) is connected by way of a bonding agent (52) to the substrate (53) of the sensor chip (18).

23. A probe according to claim 22 **characterised in that** the contact track (51) is applied by means of screen printing.

24. A probe according to claim 22 **characterised in that** the bonding agent (52) is applied by means of screen printing.

25. A probe according to one of claims 22 to 24 **characterised in that** the contact track (51) has platinum.

26. A probe according to one of claims 22 to 24 **characterised in that** the bonding agent (52) besides platinum also has glass and/or ceramic.

## Revendications

1. Sonde de mesure (10) pour un gaz à mesurer, comprenant un corps métallique (11), une plaquette de capteur (18) disposée à l'intérieur et qui présente l'élément capteur (19) dans sa première région terminale dirigée vers une ouverture d'entrée (12) du corps (11) pour le gaz à mesurer, et une connexion électrique prévue à son autre extrémité et connectée électriquement à l'élément capteur (19), **caractérisée en ce que** la plaquette de capteur (18) comprend de la céramique en tant que substrat (53), **en ce qu'**il est prévu sur ce dernier, sous la forme d'une couche, au moins une piste de contact (51) qui mène à l'élément capteur (19) et qui est conductrice de l'électricité, et **en ce que** la connexion électrique et mécanique avec la piste de contact (51) est réalisée par soudage au laser direct, opérant par force, d'au moins un fil de connexion (40) sur la piste de contact (51).

2. Sonde de mesure (10) pour un gaz à mesurer, comprenant un corps métallique (11), une plaquette de capteur (18) disposée à l'intérieur et qui présente l'élément capteur (19) dans sa première région terminale dirigée vers une ouverture d'entrée (12) du corps (11) pour le gaz à mesurer, et une connexion électrique prévue à son autre extrémité et connectée électriquement à l'élément capteur (19), **caractérisée en ce que** la plaquette de capteur (18) comprend de la céramique en tant que substrat (53), **en ce qu'**il est prévu sur ce dernier, sous la forme d'une couche, au moins une piste de contact (51) qui mène à l'élément capteur (19) et qui est conductrice de l'électricité, et **en ce que** la connexion électrique et mécanique avec la piste de contact (51) est réalisée indirectement, avec liaison opérant par force, par soudage au laser d'une cosse (50), un fil de connexion (40) étant soudé ou brasé à la cosse (50) et ce fil étant soudé au laser à la piste de contact (51) de la plaquette de capteur (18).

3. Sonde selon l'une des revendications 1 ou 2, **caractérisée en ce que** la sonde présente une cosse (50) et **en ce que** le conducteur intérieur (40) est soudé ou brasé à la cosse (50) et que cette dernière est soudée au laser à la piste de contact (51) de la plaquette de capteur (18).

4. Sonde selon l'une des revendications 1 à 3, **caractérisée en ce que** la cosse (50) conductrice de l'électricité, qui emboîte latéralement les deux côtés de la plaquette de capteur (18), est appuyée sur une des pistes de contact (51) de la plaquette de capteur (18) et est soudée au laser dans la région de cette piste de contact.

5. Sonde selon l'une des revendications 1 à 4, **caractérisée en ce que** la cosse (50) comprend deux branches élastiques (55, 56) qui sont appliquées à plat au moins dans une région partielle de la piste de contact (51) et sont reliées à cette dernière dans cette région d'appui à plat par soudage au laser.

6. Sonde selon l'une des revendications 1 ou 5, **caractérisée en ce que** chaque cosse (50) comprend une queue de connexion (41) qui s'étend vers l'ouverture d'insertion et présente de préférence un contre-coude en forme de U (54) auquel est soudé un des fils de connexion du conducteur intérieur (40) de la ligne (13) de la gaine métallique.

7. Sonde selon l'une des revendications 1 ou 6, **caractérisée en ce que** la plaquette de capteur (18), qui est de préférence allongée ou plane, comprend entre l'élément capteur (19) et l'ouverture d'insertion, un élément de soutien (20, 30) qui le soutient uniquement dans la direction perpendiculaire à l'extension longitudinale de la plaquette de capteur (18).

8. Sonde selon la revendication 7, **caractérisée en ce que** l'élément de soutien est réalisé sous la forme d'un entrelaçage de fil métallique (20), de préférence préformé, appliqué sur la plaquette de capteur (18).

9. Sonde selon l'une des revendications 1 à 8, **caractérisée en ce que** le corps métallique est constitué par un tube cylindrique (11) dont l'une des extrémités présente l'ouverture d'entrée (12) pour le gaz d'échappement qui doit être mesuré au moyen de l'élément capteur (19) et dont l'autre extrémité ouverte constitue une ouverture d'insertion pour la ligne à gaine métallique (13) insérée et soudée ou brasée à cet endroit.

10. Sonde selon la revendication 9, **caractérisée en ce qu'**une bague métallique (14) est posée sur la ligne à gaine métallique (13), ainsi que brasée ou soudée dans cet état, et **en ce qu'**à son tour, cette bague est brasée ou soudée au tube (11).

11. Sonde selon l'une des revendications 1 à 10, **caractérisée en ce que** la plaquette de capteur (18) présente sur un de ses côtés une piste de contact constituée par une métallisation (24) pour la connexion mécanique, et à laquelle au moins une petite patte (22) d'une partie de maintien (21), qui s'étend sensiblement perpendiculairement à cette piste, est soudée par soudage au laser au moyen de ses pieds (23) disposés à l'extrémité des petites pattes.

12. Sonde selon l'une des revendications 1 à 11, **caractérisée en ce que** la partie de maintien s'étend selon la direction du tube (11) et **en ce que** les petites pattes (22) font saillie transversalement, de préférence perpendiculairement, sur la partie de maintien (21).

13. Sonde selon la revendication 9, 10, 11 ou 12, **caractérisée en ce que** la partie de maintien (30) s'étend selon la direction du tube (11) et est fixée, de préférence soudée, au côté intérieur du tube (11), par son extrémité la plus extérieure (33) avec interposition d'un pli (31) en forme de V (Fig. 3).

14. Sonde selon la revendication 13, **caractérisée en ce que** le pli (31) en forme de V est disposé entre l'élément capteur proprement dit (19) et l'ouverture d'entrée (12).

15. Sonde selon l'une des revendications 1 à 14, **caractérisée en ce qu'**au moins une des petites pattes (22) est assemblée, de préférence soudée, par l'intermédiaire d'une liaison (26), à la gaine métallique de la ligne à gaine métallique (13) ou à la bague (14) au moyen d'une partie enveloppante (25).

16. Sonde selon l'une des revendications 1 à 15, **caractérisée en ce que** les petites pattes (22) emboîtent au moins partiellement le bord de la plaquette de capteur (18) et/ou s'engagent dans des évidements ménagés dans la région du bord de cette plaquette (18).

17. Sonde selon l'une des revendications 1 à 16, **caractérisée en ce qu'**au moins une des petites pattes (22) est coudée deux fois à angle droit en direction de l'autre côté de la plaquette de capteur (18) qui ne présente pas la métallisation (24).

18. Sonde selon l'une des revendications 1 à 17, **caractérisée en ce que** les différents fils électriques de connexion du conducteur intérieur (40) de la ligne à gaine métallique (13) de la connexion électrique sont reliés entre eux dans l'espace intérieur du corps qui est exposé aux gaz d'échappement à mesurer.

19. Sonde selon la revendication 18, **caractérisée en ce que** les fils électriques de connexion du conducteur intérieur (40) sont soudés directement sur la plaquette de capteur par soudage au laser, éventuellement au moyen d'un coude préformé (43) servant de compensation de longueur et/ou de détente des tractions.

20. Sonde selon l'une des revendications 1 à 19, **caractérisée en ce que** les fils de connexion (40) sont connectés aux queues de contact (41) au moyen d'une bague d'assemblage (42).

21. Sonde selon l'une des revendications 1 à 20, **caractérisée en ce que** les fils de connexion (40) sont préformés pour leur connexion, par exemple préformés en formant un arc (43).

22. Sonde selon l'une des revendications 1 à 21, **caractérisée en ce que** la piste de contact (51) est assemblée au substrat (53) de la plaquette de capteur (18) au moyen d'un promoteur d'adhérence (52).

23. Sonde selon la revendication 22, **caractérisée en ce que** la piste de contact (51) est appliquée par sérigraphie.

24. Sonde selon la revendication 22, **caractérisée en ce que** le promoteur d'adhérence (52) est appliquée par sérigraphie.

25. Sonde selon l'une des revendications 22 à 24, **caractérisée en ce que** la piste de contact (51) comprend du platine.

26. Sonde selon l'une des revendications 22 à 24, **caractérisée en ce que** le promoteur d'adhérence (52) comprend, en dehors du platine, également du verre et/ou une céramique.
